# EUROPEAN PATENT APPLICATION

(11) **EP 0 998 890 A1**
(43) Date of publication of application: **10.05.2000**
(21) Application number: 98124198.7
(22) Date of filing: 21.12.1998
(51) Int. Cl.: A61F 13/15

(54) **Wrapped sanitary napkin with improved flap disposition**

(30) Priority: 02.11.1998 EP 98120610
(71) Applicant: THE PROCTER & GAMBLE COMPANY, Cincinnati, Ohio 45202 (US)
(72) Inventor: Hirsch, Uwe Thomas Michael Horst, 64347 Griesheim (DE); Borbach, Markus, 65812 Bad Soden (DE)
(74) Representative: Kohol, Sonia

(57) **Abstract**

The present invention relates to sanitary napkins, and particularly sanitary napkins having side flaps (28). More particularly, this invention is directed to the packaging and handling of such sanitary napkins prior to the first use by the wearer. Claimed and described is a sanitary napkin (20) comprising side flaps (28). The sanitary napkin (20) is individually wrapped and provided in a certain prior to use configuration which is maintained by disposition aides.

## Description

### Field of the invention

The present invention relates to sanitary napkins, and particularly sanitary napkins having side flaps. More particularly, this invention is directed to the packaging, individual wrapping, handling and the disposition of the side flaps of such sanitary napkins prior to the use by the wearer.

### Background of the invention

Sanitary napkins used to collect vaginal discharges, and in particular embodiments having side flaps, are well known in the art and are products of mass production. For example US 4,589,876 and US 4,687,478 disclose preferred sanitary napkins with side flaps, which improve soiling protection and the secure installation in an undergarment.

For shipment and sale sanitary napkins are typically packaged. Inter alia for economical reasons a small size of any such package (comprising at least one sanitary napkin) is desirable. To this benefit the side flaps can be folded over the backsheet as taught in US 4,701,178 or they can be folded over the topsheet as taught in WO 91/16873.

Sanitary napkins, independently from other packaging means, may be provided with a wrapping material to form an individual package. Such individually packaged sanitary napkin are disclosed, e.g. in WO 91/18574. Typically the wrapping material used for such embodiments is a thin plastic sheet, e.g. made of polyethylene. Such wrapped sanitary napkin are often carried individually by the user, for example in a handbag. In such circumstances of transport it may easily happen that the wrapping material suffers damage, for example when in contact with other items carried in a handbag.

While it may be acceptable to a user when some portions, e.g. close to a side margin, of the sanitary napkin suffer damage or are affected in their hygienic state, it appears typically unacceptable to a user if the central portions of the sanitary napkin are affected in such manner, namely those portions which will come in contact with the wearer's genitals, herein referred to as contact portions.

Thus, when a sanitary napkin is provided with a thin plastic wrapping material, there is a need for the additional protection of the contact portions. This additional protection should be particularly effective with regard to mechanical impact on the sanitary napkin.

To achieve a better mechanical protection of in particular the contact portions would typically require the utilisation of a different wrapping material. For example, the utilisation of a thicker wrapping material may yield better protection against mechanically induced damage. However, this would induce higher material costs. Moreover, this would result in an increased amount of packaging material to dispose of, which is not desirable for ecological reasons.

WO 91/16873 discloses an approach to protection of a sanitary napkin with side flaps prior to use of the sanitary napkin. By folding of the side flaps over the topsheet protection of the overlaid parts of the topsheet is achieved. The side flaps are held in that overlaying position for example by the use of a bridging release paper, which is in contact with adhesive portions on both side flaps.

However, protection of the sanitary napkin solely by the prior to use configuration of the side flaps will often appear insufficient. Typically, when folded over the topsheet, the side flaps cover only portions of the topsheet and do not cover other parts of the sanitary napkin. Furthermore, the side flaps provide only limited protection for example against the impact of liquids or dust.

While the preferred prior to use configuration comprises the folding of the side flaps over the topsheet, for some sanitary napkin embodiments the desired protection effect may be achieved by prior to use configuration, e.g. those comprising folding over the backsheet.

The present invention further improves the handling convenience in the application of a sanitary napkin. Typically the backsheet of a sanitary napkin and the backsheets of the side flaps comprise adhesive portions for the attachment of the sanitary napkin and the attachment of the side flaps to an undergarment, which prior to use are covered with a number of release papers. The user typically then removes the release means for the panty fastening adhesive first, places the article onto the undergarment and then removes the release means for the side flap adhesive in order to attach the side flaps to the undergarment.

A problem with the presence of such adhesives on the side flaps is that the side flaps tend to stick onto themselves, onto other parts of the sanitary napkin, and/or the skin of the wearer etc. while and after the sanitary napkin is being unpacked by the wearer, in particular after the release means covering the side flaps adhesive has been removed.

When unpacking a wrapped sanitary napkin and applying it to the undergarment, the wearer will typically have to remove and dispose of the wrapping material, the release paper covering the adhesive on the backsheet of the sanitary napkin and one or two release papers covering the adhesive portion of each side flap. It has been recognised in the recent prior art that the need to remove and dispose of several separate pieces of packaging material is often inconvenient, since the installation of a sanitary napkin occurs on a routine basis and under various circumstances.

Various recent attempts have been undertaken to achieve a more convenient removal and disposal of the packaging material including the release papers. EP 0 647 128 describes an approach whereby the side flaps themselves are folded along a longitudinal line, so that the adhesive on the backsheet of the side flaps lies in a fold and does not need a release paper. Similarly EP 0 675 704 describes an alternative folding of the side flaps prior to use, which also avoids the use of a separate release means. A shortcoming of such embodiments, however, is that in some executions the side flaps may not be suitable for such folding and the provision of an appriopriate release means for the side flap adhesive is not easily achieved. Furthermore the inclusion of the process step of additional folding of the wings in the mass production of such sanitary napkin induces undesirable costs.

WO 91/18574 describes a way of packaging a sanitary napkin which allows the adhesive areas both on the backsheet of the main sanitary napkin body and the backsheet of the side flaps to be covered by the wrapping material, thus avoiding the use of all additional release means. This way of packaging can be applied to a sanitary napkin with various side flap dispositions. EPO 0 750 896 describes the attachment of two release sheets to the wrapping material, giving the benefit of only having to dispose of one joined piece of packaging material.

A shortcoming of the approaches described in WO 91/18574 and in EPO 0 750 896 is that the side flaps are not held in place after removing the release means covering the side flaps adhesive. Some consumers may not consider the benefit of a one step removal and disposal of the wrapping material to counterbalance the problem of premature sticking of the side flaps. In particular, when large, soft side flaps are used, as generally taught in US 4,917,697 and WO 94/27541, premature sticking of the side flaps in particular onto themselves has been encountered and found particularly acute hence the holding the side flaps in their prior to use position is still very desirable.

WO 91/16873 discloses a sanitary napkin embodiment where the side flaps are held in the prior to use position by covering each adhesive portion of each side flap with a unitary release means bridging the side flaps. This embodiment has the disadvantage of involving a separate release means to cover the side flap adhesives, as discussed below.

It has now been found, that an appropriate prior to use configuration of the side flaps in combination with the use of a thin wrapping material provides an improved protection at low cost and in an ecological manner. It has now further been found, that an individually wrapped sanitary napkin can be provided with a disposition aide for the side flaps, which allows maintenance of the side flaps in the prior to use configuration independently from the removal of the wrapping material. Moreover, this disposition aide does not comprise pieces of material, such as release means, which require separate disposal. In avoiding the use of such pieces of material the present disposition aide also allows the cost effective production of sanitary napkins.

It is hence an objective of the present invention to provide a protective individual packaging of a sanitary napkin.

It is a further objective of the present invention to provide such packaging with the utilisation of a minimal amount of packaging material in a ecological and cost effective manner.

It is a further objective of the present invention to provide a means for holding the side flaps in a particular position prior to use.

It is also an objective of the present invention to provide such a means comprising only a minimal amount of material.

It is a further objective to provide a sanitary napkin which can be packaged using a unitary piece of wrapping material and to avoid the separate disposal of additional release means covering adhesive portions.

### Summary of the invention

The present invention relates to sanitary napkins, and particularly sanitary napkins having side flaps (28). More particularly, this invention is directed to the packaging of such sanitary napkins prior to the first use by the wearer. Claimed and described is a sanitary napkin (20) comprising side flaps (28). The sanitary napkin (20) is individually wrapped and provided in a certain prior to use configuration which is maintained by disposition aides. One preferred disposition aide is a topsheet contact adhesive (50), which maintains adhesive contact between the topsheet (22) and the side flaps (28). Another preferred disposition aide is a flap contact adhesive (52), which maintains adhesive contact between the side flaps (28).

### Brief description of the drawings

It is believed that the invention will be better understood from the foregoing description in conjunction with the accompanying drawings in which:
Figure 1 is a top plan view of a sanitary napkin (20) in a preferred prior to use configuration.
Figure 2 is a top plan view of a sanitary napkin (20) in the preferred prior to use configuration depicted in Figure 1 comprising a preferred disposition aide, which comprises a topsheet contact adhesive in a certain disposition.
Figure 3 is a top plan view of a sanitary napkin (20) in the preferred prior to use configuration depicted in Figure 1 comprising a preferred disposition aide, which comprises a topsheet contact adhesive in a different disposition.
Figure 4 is a top plan view of a sanitary napkin (20) comprising overlapping side flaps and a preferred disposition aide, which comprises a flap contact adhesive.
Figure 5 is a vertical sectional view of a sanitary napkin with a C-fold wrapping material.

### Description of the invention

While the term sanitary napkin is used throughout the description the present invention is equally applicable to panty liners, light incontinence guards and other absorbent articles.

### Terms of description

To allow a more detailed and clear description of the present invention, in the following paragraphs a number of terms, as used herein, will be defined.

The term "longitudinal", as used herein, refers to an imaginary line, axis or direction of the sanitary napkin (20), which line, axis or direction is typically centred between the side margins of the napkin and is generally aligned with the vertical plane which bisects a standing wearer into left and right body halves. The term "lateral" refers to an imaginary line, axis or direction generally orthogonal the longitudinal direction and within the plane of the sanitary napkin (20), and is generally aligned sideways relative to the wearer.

The "horizontal plane", as used herein, is the plane which contains a longitudinal and a transversal axis. The vertical direction is perpendicular to that horizontal plane.

### The sanitary napkin as a whole

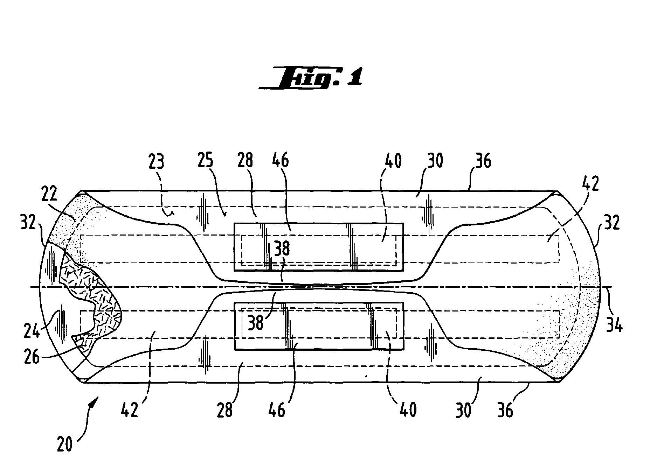

The invention relates to a sanitary napkin (20) as shown in Figure 1. The sanitary napkin (20) comprises a topsheet (22), a backsheet (24), an absorbent core (26) and side flaps (28) and has a longitudinal side margin (30) and a lateral side margin (32). The perimeter of the sanitary napkin (20) is defined by two longitudinal side margins (30) and two lateral side margins (32).

### The topsheet

The topsheet (22) is compliant, soft feeling, and non-irritating to the wearer's skin. The topsheet (22) also can have elastic characteristics allowing it to be stretched in one or two directions in portions of the topsheet (22) or throughout its extension. Further, the topsheet (22) is fluid pervious permitting fluids (e.g., menses and/or urine) to readily penetrate through its thickness. A suitable topsheet (22) can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearers skin. Thus, the surface of the formed film that is in contact with the body remains dry, thereby reducing body soiling and creating a more comfortable feel for the wearer. Suitable formed films are described in US 3,929,135; US 4,324,246; US 4,342,314; US 4,463,045; and US 5,006,394. Particularly preferred micro apertured formed film topsheets are disclosed in US 4,609,518 and US 4,629,643. A preferred topsheet (22) for the present invention comprises the formed film described in one or more of the above patents and marketed on sanitary napkins by The Procter & Gamble Company of Cincinnati, Ohio as "DRI-WEAVE".

Topsheets not having a homogeneous distribution of liquid passage ways but only a portion of the topsheet (22) comprising liquid passage ways are also contemplated by the present invention. Typically such topsheets would have the liquid passage ways oriented such that they result in a centrally permeable and peripherally impermeable topsheet (22) for liquids.

The body surface of the formed film topsheet (22) can be hydrophilic so as to help liquid to transfer though the topsheet (22) faster than if the body surface was not hydrophilic. In a preferred embodiment, surfactant is incorporated into the polymeric materials of the formed film topsheet (22) such as is described in PCT-publication WO 93/09741. Alternatively, the body surface of the topsheet (22) can be made hydrophilic by treating it with a surfactant such as is described in US 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet (22) typically extends across the whole of the absorbent structure and outside the area coextensive with the absorbent structure. The topsheet (22) can extend and form part or all of the preferred side flaps.

When referring to the topsheet (22) a multi layer structure or a mono layer structure is contemplated. The hybrid topsheet (22) mentioned above is such a multi layer design but other multi layer topsheets such as primary and secondary topsheet (22) designs are also considered.

### Absorbent core

According to the present invention the absorbent cores suitable for use in herein may be selected from any of the absorbent cores or core system known in the art. As used herein the term absorbent core (26) refers to any material or multiple material layers whose primary function is to absorb, store and distribute fluid.

According to the present invention, the absorbent core (26) can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a. Primary/Secondary Fluid Distribution Layer

One optional component of the absorbent core (26) according to the present invention is a primary fluid distribution layer and a secondary fluid distribution layer. The primary distribution layer typically underlies the topsheet (22) and is in fluid communication therewith. The topsheet (22) transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b. Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c. Optional Fibrous ("Dusting") Layer

An optional component for inclusion in the absorbent core (26) according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core (26). Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d. Other Optional Components of the absorbent structure

The absorbent core (26) according to the present invention can include other optional components normally present in absorbent webs. For example, a reinforcing scrim can be positioned within the respective layers, or between the respective layers, of the absorbent core (26). Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core (26) according to the invention and preferably is provided close to or as part off the primary or secondary fluid distribution layer are odor control agents.

### Backsheet

The backsheet (24) primarily prevents the extrudes absorbed and contained in the absorbent structure from wetting articles that contact the absorbent product such as underpants, pants, pyjamas and undergarments. The backsheet (24) is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet (24) also can have elastic characteristics allowing it to stretch in one or two directions.

The backsheet (24) typically extends across the whole of the absorbent structure and can extend into and form part of or all of the preferred sideflaps, side wrapping elements or wings.

The backsheet (24) can comprise a woven or nonwoven material, polymeric films such as thermoplastic films of polyethylene or polypropylene, or composite materials such as a film-coated nonwoven material. Preferably, the backsheet (24) is a polyethylene film having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet (24) is preferably embossed and/or matt finished to provide a more clothlike appearance.

Further, the backsheet (24) can permit vapours to escape from the absorbent structure, i.e. be breathable, while still preventing extrudates from passing through the backsheet (24). Also breathable backsheets comprising several layers, e.g. film plus non-woven structures, can be used. Such backsheets thus comprise at least one gas permeable layer. Suitable gas permeable layers include 2-dimensional, planar micro and macro-porous films, macroscopically expanded films, formed apertured films and monolithic films. The apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet (24) may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1 mm, preferably less than 0.5 mm, wherein the apertures have an average uniform diameter along their length and which do not protrude out of the plane of the layer. The apertured materials for use as a backsheet (24) in the present invention may be produced using any of the methods known in the art such as described in EPO 293 482 and the references therein. In addition the dimensions of the apertures produced by this method may be increased by applying a force across the plane of the backsheet (24) layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane™ available from B.F. Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet (24) component comprises a microporous film and an apertured formed film or a microporous and a hydrophobic woven or nonwoven material.

The sanitary napkin (20) preferably also has fasteners for securing the sanitary napkin (20) in place in a wearer's undergarment. The fasteners used with the sanitary napkin (20) are not limited to adhesive fasteners. Any suitable type of fastener known in the art can be used for this purpose. For example, the sanitary napkin (20) could be secured in place in a wearer's undergarment by mechanical fasteners, such as VELCRO™ or by a combination of adhesive and mechanical fasteners. For simplicity, however, the fasteners will be described in terms of adhesive fasteners and these fasteners are preferably pressure sensitive adhesive fasteners. Suitable pressure sensitive adhesive fasteners are described in greater detail in US 4,917,697.

The panty fastening adhesive can be provided in any suitable configuration. Typically the panty fastening adhesive is positioned on the backsheet of the sanitary napkin. In a preferred embodiment, the panty fastening adhesive is provided in the form of a longitudinally oriented strip of adhesive that is centred about the principal longitudinal centreline (34). The panty fastening adhesive provides an adhesive attachment means for securing the main body portion of the sanitary napkin (20) in the crotch portion of a panty.

### The side flaps

The sanitary napkin (20) comprises at least one side flap (28) extending from each of the longitudinal side margins (30) of the sanitary napkin (20). The side flaps (28) have a proximal end (36) which is typically coincident with the juncture of attachment of the side flap (28) to the longitudinal side margin (30) of the sanitary napkin (20). Alternatively, the proximal end (36) of the side flap (28) may be joined to the sanitary napkin (20) at another location, remote from but juxtaposed with the longitudinal side margin (30).

The side flaps (28) extend laterally outwardly from the sanitary napkin (20) and terminate at a distal end (38) which represents the portion of the side flaps (28) furthest from the longitudinal side margins (30) of the sanitary napkin (20). The distal ends (38) of the side flaps (28) are directed away from the longitudinal centreline (34) and central portion of the sanitary napkin (20). As used herein the phrase "central portion" refers to that part of the sanitary napkin (20) intermediate, particularly laterally intermediate, and defined by the proximal ends (36) of the side flaps (28). The side flaps (28) may be of any shape desired, with a preferred shape being shown in Figure 1.

The side flaps (28) may be comprised of an integral and contiguous extension of other parts of the sanitary napkin (20), for example the topsheet (22), the backsheet (24), or a laminate of both (22) and (24) and may also comprise absorbent material. Alternatively, the side flaps (28) may be made of a separate and independent piece or pieces of material joined to the longitudinal side margins (30) of the sanitary napkin (20). In both cases each side flap (28) has one face generally coextensive of the topsheet (22), referred to as topsheet (23) of the side flap (28), and a mutually opposed face generally coextensive of the backsheet (24), referred to as backsheet (25) of the side flap (28).

The side flaps (28) preferably have a means (40) for attaching one face of the side flap (28) to the wearer's undergarment or to the other side flap (28). The attachment means (40) may be a mechanical fastener or, preferably, an adhesive fastener, most preferably a pressure sensitive adhesive. If pressure sensitive adhesive is selected, it should be disposed on the backsheet (25) of the side flaps (28) so that when the side flaps (28) are wrapped around the crotch portion of the wearer's undergarment, the adhesive will face the outside of the wearer's undergarment. A generally rectangular patch of adhesive on each side flap (28), about 68 mm x 14 mm in size, works well. Suitable pressure sensitive adhesive is sold by the Anchor Continental, Inc., 3 Sigma Division of Covington, Ohio. Prior to use such an adhesive (40) may be covered by a dedicated release strip (46). Prior to use such an adhesive (40) may be covered by a dedicated release strip (46).

The present invention provides a disposition aide which maintains the side flaps (28) of a sanitary napkin (20) in a certain prior to use configuration. Such a prior to use configuration is chosen for packaging, shipment and sale. The term "prior to use configuration", as used herein, relates only to the disposition of the side flaps (28) with regard to the sanitary napkin (20) and not to the configuration of the sanitary napkin (20) as a whole when packaged, shipped or sold. The term "use" as used herein refers to the use of a sanitary napkin commencing with the installation of the sanitary napkin to an undergarment. The handling and unwrapping of a sanitary napkin is not considered part of its use.

The most preferred prior to use configuration is shown in Figure 1. In this prior to use configuration the side flaps (28) are folded over the topsheet (22). In this configuration the topsheet (23) of the side flaps (28) and the topsheet (22) of the sanitary napkin (20) are facing each other and typically at least a part of the topsheet (23) of the side flaps (28) is in direct contact with a part of the topsheet (22). The side flaps (28) are preferably folded, more preferably along longitudinal lines, most preferably along the longitudinal lines generally coincident the proximal edges (36) of the side flaps (28), preferably so that the maximum surface area of the topsheet (22) is covered by the side flaps (28). In this configuration the distal ends (38) of the side flaps (28) are in closer spatial distance than the proximal ends (36) of the side flaps (28). In one preferred prior to use configuration the side flaps do not overlap each other (i.e. the distal ends of the side flaps are not in contact with each other), but prior to use configurations with overlapping side flaps are also preferred. The preferred configurations provide a large area of the topsheet (22) which is protectively overlaid by the side flaps (28), so that a sanitary and clean appearance of the topsheet (22) is maintained. Particularly, the area of the topsheet (22) which is overlaid is typically in registry with the wearer's genitals.

Another, less preferred prior to use configuration involves the folding of the side flaps (28) over the backsheet (24) of the sanitary napkin (20). In this configuration the backsheet (25) of the side flaps (28) and the backsheet (24) are facing each other and typically at least a part of the backsheet (25) of the side flaps (28) is in contact with a part of the backsheet (24). The side flaps (28) are preferably folded, more preferably along longitudinal lines, most preferably along the longitudinal lines generally coincident the proximal edges (36) of the side flaps (28), preferably so that the maximum surface area of the backsheet (24) is covered by the side flaps (28). In this configuration the distal ends (38) of the side flaps (28) are in closer spatial distance then the proximal ends (36) of the side flaps (28). Other preferred prior to use configurations may involve a different folding of the side flaps (28), e.g. along more than one longitudinal line.

The present invention in particular with respect to Figures 1 to 4 is described for preferred embodiments of the disposition aide for a specific prior to use configuration, wherein the side flaps (28) are folded over the topsheet (22). However, the above described alternative embodiments are equally applicable and the described disposition aide can also be used in combination with other prior to use configuration.

The prior to use configuration is typically only changed for the first time for the installation of the sanitary napkin (20) to an undergarment, which for a conventional sanitary napkin, comprising side flaps folded over the topsheet prior to use, comprises the following handling steps: unwrapping of the sanitary napkin (20) which is typically provided in a wrapping material (60), removal and then disposal of release means, if present, for the panty fastening adhesive, installation of the sanitary napkin (20) to an undergarment, removal and then disposal of release means, if present, for the side flap adhesive (40), unfolding and application of the side flaps (28) to an undergarment. During this operation it is highly desirable to maintain the side flaps in the prior to use configuration until application, so that they do not unintentionally adhere to undesirable surfaces.

The term disposition aide as used herein is to be understood as a means which supports the disposition of the side flaps (28) in their prior to use configuration.

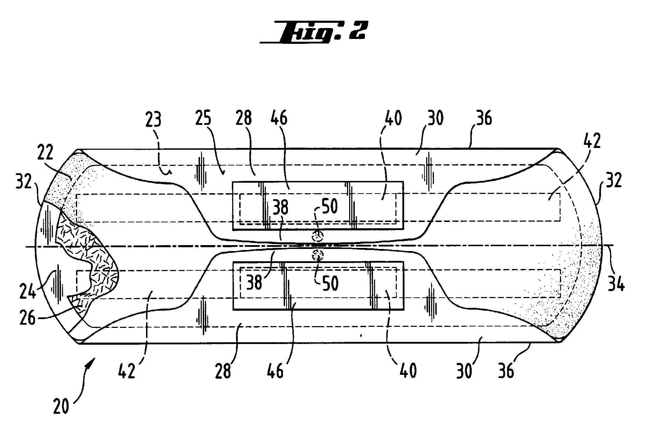

Figure 2 depicts one preferred embodiment of a disposition aide. The disposition aide comprises an adhesive, which is in adhesive contact with the topsheet (23) of the side flaps (28) and the topsheet (22) of the sanitary napkin (20), referred to as topsheet contact adhesive (50). The unfolding of the side flaps (28) for the installation of the sanitary napkin (20) in an undergarment requires breaking of the adhesive contact. Hence the topsheet contact adhesive (50) serves to maintain the side flaps (28) in the prior to use configuration until application to an undergarment.

In the preferred embodiment of the present invention shown in Figure 2 the topsheet contact adhesive (50) is disposed adjacent to the distal ends (38) of the side flaps (28). The surface area of the topsheet contact adhesive (50) is preferably the same at the topsheet (22) contacting surface and at the side flap (28) contacting surface and is preferably chosen to be less then 50 mm², preferably less then 10 mm². This disposition of the topsheet contact adhesive (50) results in a small portion of the topsheet (22) which is contacted by the topsheet contact adhesive (50). This small contact portion will reduce any effects some adhesives may have on the topsheet. The disposition of the topsheet contact adhesive (50) adjacent to the distal ends (38) of the side flaps (28) increases the force required to unfold the side flaps (28) when compared to a disposition adjacent to the proximal ends (36) of the side flaps (28) due to leverage effects. Thus, a less aggressive adhesive can be used.

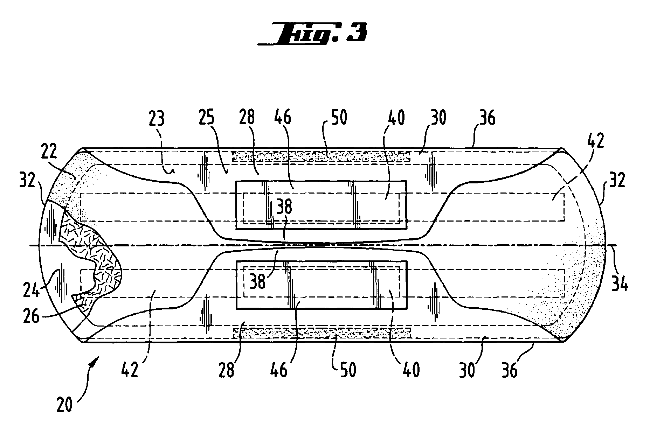

Another preferred embodiment of the present invention comprises a topsheet contact adhesive (50) of a different disposition and is shown in Figure 3. In this embodiment the topsheet contact adhesive (50) is disposed adjacent to the proximal ends (36) of the side flaps (28). Preferably an area of the topsheet contact adhesive (50) of at least 10 mm², more preferably 20 mm² is chosen. The area of the topsheet contact adhesive (50) may extend over the whole length of the sanitary napkin (20) in the longitudinal direction or may be confined to a longitudinally centered area adjacent to the side flaps (28), as shown in Figure 3. The width of the area of the topsheet contact adhesive (50) is typically 1 to 10 mm. This disposition of topsheet contact adhesive (50) avoids the contact of the adhesive with the areas of the topsheet which are likely to come in contact with a wearer's genitals. Whilst adhesives are available which are suitable for such contact, some wearers may prefer the contact portions of the topsheet (22) not to have been in contact with any adhesive. A larger area of the topsheet contact adhesive (50) allows to compensate for the leverage effect which is involved in unfolding the side flaps (28) when the topsheet contact adhesive (50) is disposed adjacent to the proximal ends (36) of the side flaps (28). Furthermore, a large area of the topsheet contact adhesive (50) allows the use of any aggressive adhesive to be avoided which may for example affect the performance of the topsheet (22) or the performance of the absorbent core (26).

An adhesive used as topsheet contact adhesive (50) for the embodiments described above preferably has the following properties: The adhesive should maintain sufficiently strong adhesive contact with the side flaps (28) and the topsheet (22) as long as the sanitary napkin (20) is intended to stay in its prior to use configuration. More particularly, the adhesive should maintain sufficiently strong adhesive contact with the side flaps (28) while a release means covering the side flap adhesive (40) is being removed. On the other hand, the adhesive should be so chosen that the unfolding of the side flaps (28) as described above is conveniently possible. Thus, when the side flaps (28) are handled, typically by the wearer's fingers, and force is applied with the intention to unfold the side flaps (28) the adhesive contact to at least one side flap (28) should not prevent convenient separation. Furthermore the adhesive preferably does not remain sticky and after its application to the sanitary napkin (20), in particular at the point in time when the sanitary napkin (20) is sold. A suitable adhesive is produced by Atofindley of Elf Atochem (France) under the name H1322.

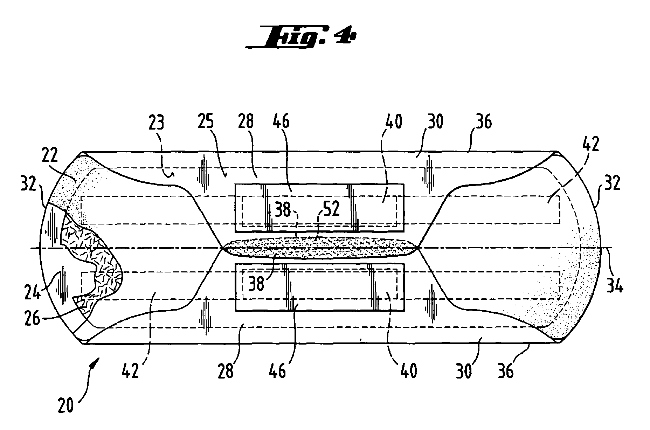

Another preferred disposition aide of the present invention is depicted in Figure 4. This disposition aide can be utilised with a sanitary napkin which comprises at least two side flaps (28) which are overlapping in their prior to use configuration. The disposition aide comprises an adhesive, referred to as flap contact adhesive (52).

In the following, this embodiment will be described with reference to a sanitary napkin (20) having two side flaps (28), however, the present invention is equally applicable for a sanitary napkin (20) comprising more side flaps (28). Overlapping as used herein with regard to the side flaps (28) describes a prior to use configuration in which the topsheet (23) of a first side flap (28) is in contact with the backsheet (25) of a second side flap (28). The term "contact" as used herein comprises a contact an adhesive.

The disposition aide which is comprised by the present invention is an adhesive disposed on the topsheet (23) of a first side flap (28) which is in adhesive contact with the backsheet (25) of a second side flap (28). The area of the flap contact adhesive (52) largely depends on the specific sanitary napkin embodiment and the specific prior to use configuration. The area depends in particular on the area of the overlap between the two side flaps (28) and on the adhesive chosen as flap contact adhesive (52). The properties of the flap contact adhesive (52) can be chosen as described for the topsheet contact adhesive (50).

The disposition aides described herein may also be advantageously used in combination. For example a sanitary napkin comprising at least two side flaps (28), which are overlapping, may comprise a topsheet contact adhesive (50) and a flap contact adhesive (52).

While the described disposition aides can be used with any sanitary napkin (20) embodiment, the present invention provides an additional benefit when used in combination with sanitary napkins, which are folded and packaged to provide a single unit. Preferably the sanitary napkin of the present invention is wrapped by a single or multiple wrapping material in any manner. For example, the sanitary napkin (20) may be folded prior to wrapping or alternatively after wrapping and together (i.e. as one unit) with the wrapping material. Alternatively the sanitary napkin maybe partly folded prior to wrapping and partly folded after wrapping together with the wrapping material. Preferably the sanitary napkin and the wrapping material are folded to provide a small and convenient package, as e.g. disclosed in WO 91/18574.

The wrapping material (60) can be made from any suitable material. The wrapping material (60) is preferably manufactured from a thin flexible material which is liquid impermeable so that the wrapping material (60) will be suitable for wrapping and disposing of a used sanitary napkin (20). For example, polyethylene films have been found to work well.

The wrapping material (60) can be provided with an optional release component, such as release paper or preferably a release coating so that the wrapping material (60) will release from the panty fastening adhesive (42) and side flap adhesive (40) when the wearer removes the sanitary napkin (20) from the wrapping material (60). If a separate release paper is used, it can comprise any suitable material known in the art for this purpose, such as coated papers. Suitable release papers are described in US 4,917,697. Such a release paper can be laminated to the inside surface of the wrapping material (60). If a release coating is used, the coating can be applied directly to the inside surface of the wrapping material (60). Such a coating can comprise any material known in the art for this purpose, with silicone coatings being preferred. If a coating is used, the coating may be provided by coating only that zone of the wrapping material (60) which will substantially contact the panty fastening adhesive areas. Alternatively, the entire inside surface of the wrapping material (60) may be coated. Coating the entire inside of a wrapper is disclosed in US 5,181,610.

The present invention provides increased protection of the contact portions of the topsheet (22). In particular when only a lesser protection seems required the wrapping material (60) may be designed to comprise first and second portions.

Said first portions of the wrapping material (60) are those portions which are substantially not covering the side flaps (28) when the sanitary napkin (20) is wrapped utilising the wrapping material (60), whereas the second portions are those portions which are covering the side flaps (28). The second portions of the wrapping material (60) can then for example be thinner than the first portions since the protective effect of the second portions in combination with the protective effect of the side flaps (28) with regard to the protection of the contact portions may be considered sufficient. Physical properties other than thickness, such as resistance to tearing forces, or other properties may also be adapted, for example to achieve an economically and environmentally beneficial saving of material. Provision of a release surface such as a siliconised surface on the second portion is not considered as an adaptation of the physical properties of the second portions.

If a release means is permanently attached to the wrapping material, i.e. the release means will be removed in one part with the wrapping material when the sanitary napkin (20) is unwrapped, the release means is understood as a part of the wrapping material.

According to the present invention the wrapping material is preferably juxtaposed with the sanitary napkin (20) so that the side flaps (28) in their prior to use configuration are covered. This is particularly beneficial for embodiments where the side flaps (28) comprises an adhesive (40) for attachment to an undergarment, which is consequently covered by the wrapping material, thereby rendering the need for a separate release means redundant. Such an embodiment can be achieved by for example wrappping the wrapping material around the sanitary napkin (20) in a so called C-fold as described in WO 91/18574.

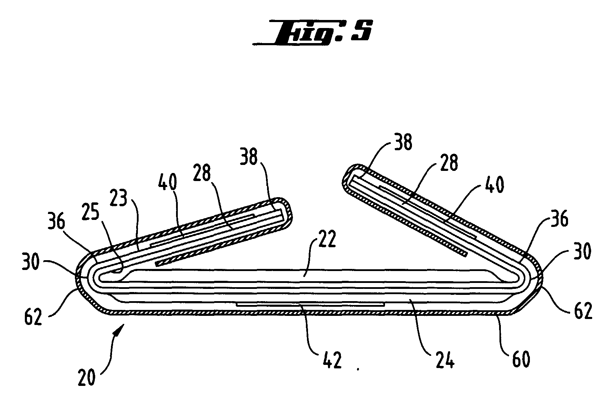

The C-fold wrapping is described with reference to Figure 5. The wrapping material (60) wraps at least one, and preferably each, longitudinal side margin (30) of the sanitary napkin (20) in a C-fold. As used herein, a "C-fold" refers to the configuration of a component which is folded over itself to provide a double thickness and may have a foreign component interposed between the layers of the folded component. As illustrated in Figure 5, it is preferred that the sanitary napkin (20) and wrapping material (60) be equivalently and symmetrically disposed and folded about the longitudinal centreline (34).

In the C-folded arrangement of Figure 5, the entire backsheet (24) and panty fastening adhesive (if present) is covered by the wrapping material (60) and a portion of the topsheet (22) juxtaposed with the longitudinal side margins (30) are also covered by the wrapping material (60).

The illustrated arrangement provides the advantage that one entire major face, particularly the face associated with the backsheet (24), the longitudinal side margins (30) of the sanitary napkin (20) and additionally a portion of the topsheet (22) and a portion of the side flaps (28) are protected by the wrapping material (60).

The wrapping material (60), in addition to C-folding around the longitudinal side margins (30) and preferably covering the side flap adhesive (40) of the sanitary napkin (20), may be fully or partially wrapped about the distal ends (38) of the side flaps (28) as shown in Figure 5. The wrapping material (60) of such a configuration will typically have a segment interposed between the side flap (28) and the topsheet (22). Wrapping around the distal ends (38) of the side flaps (28) may be only applicable for some disposition aides described herein if only parts of the wrapping material are wrapped around the distal ends (38) of the side flaps (28). Embodiments, in which the distal ends (38) of the side flaps (28) are not covered by the wrapping material are equally preferred.

Preferably the sanitary napkin and the wrapping material are folded about two laterally spaced apart fold lines to form a more handy package. Most preferably two lateral folding lines are chosen, so that the sanitary napkin and the wrapping material form an e-fold. Folding about more than two lateral folding lines has also been found beneficial in order to obtain a small package size. Such a laterally folded configuration, in particular the e-fold configuration is preferably maintained by a tab for releasable contact with the wrapping material (60) as disclosed in WO 91/18574 or in WO 94/04111.

The unwrapping of the sanitary napkin (20) provided with a wrapping material which covers the adhesive of the side flaps (28) involves peeling the wrapping material (60) off the adhesive areas. The wrapping material (60) preferably covers both, the panty fastening adhesive and the side flap (28) adhesive, so that separate release means for the side flap (28) adhesive do not need to be removed and disposed of. Preferably the wrapping material (60) can be disposed of in one piece. Hence an advantage of the present invention is that the disposition aide ensures that the side flaps remain in the prior to use configuration while the wrapping material (60) is removed. If no disposition aide is used the side flaps (28) may easily unfold and stick to the backsheet (24) of the sanitary napkin (20) or to other undesirable places during the unwrapping process, thus causing great inconvenience to the wearer when installing the sanitary napkin (20). The present invention allows the side flaps (28) to be detached and unfolded from their prior to use configuration independently from the removal of the wrapping material (60) at any time before or after the application of the sanitary napkin (20) to an undergarment.

### Glossary

- 20: sanitary napkin
- 22: topsheet
- 23: topsheet of a side flap
- 24: backsheet
- 25: backsheet of a side flap
- 26: absorbent core
- 28: side flap
- 30: longitudinal side margin
- 32: lateral side margin
- 34: longitudinal centreline
- 36: proximal ends of flaps
- 38: distal ends of flaps
- 40: side flap adhesive
- 42: panty fastening adhesive
- 46: release strip
- 50: topsheet contact adhesive
- 52: flap contact adhesive
- 54: unitary release means
- 60: wrapping material
- 62: longitudinal side margin of wrapping material

## Claims

1. A sanitary napkin (20) comprising a topsheet (22) and at least two side flaps (28) and further comprising a wrapping material (60), said side flaps (28) having a prior to use configuration and being maintained in said prior to use configuration by a disposition aide, said disposition aide being selected from:
a) a disposition aide comprising a topsheet contact adhesive (50) contacting said topsheet (22) and at least one of said side flaps (28)
b) a disposition aide to be used in combination with a prior to use configuration having overlapping side flaps (28), thereby providing an area of overlap and said disposition aide comprising a flap contact adhesive (52) disposed in said area of overlap.

2. A sanitary napkin (20) according to Claim 1 characterised in that said disposition aide which comprises said topsheet contact adhesive (50) is in contact with at least two of said side flaps (28).

3. A sanitary napkin (20) according to any one of the preceding claims, wherein said side flaps (28) each comprise a backsheet (25) and at least one of said backsheets (25) of said side flaps (28) comprises a side flap adhesive (40) and said wrapping material (60) covers said side flap adhesive (40) on said backsheet (25) of said side flap (28) in said prior to use configuration.

4. A sanitary napkin (20) according to any one of the preceding claims comprising two longitudinal side margins (30) and a backsheet (24), said backsheet (24) comprising a panty fastening adhesive (42), said wrapping material (60) comprising longitudinal side margins (62), characterised in that said wrapping material is firstly releasably affixed to said panty fastening adhesive (42) of said backsheet (24) and said wrapping material (60) is wrapped around said longitudinal side margins (30) of said sanitary napkin (20) in a C-fold.

5. A sanitary napkin (20) according to Claim 4 characterised in that said wrapping material (60) covers said side flap adhesive (40) on said backsheet (25) of each of said side flaps (28) in said prior to use configuration.

6. A sanitary napkin (20) according to any one of the preceding claims characterised in that said sanitary napkin (20) and said wrapping material (60) are folded about one or more transversal lines.

7. A sanitary napkin (20) according to Claim 7 characterised in that said wrapping material (60) comprises a tab to releasably maintain said sanitary napkin (20) and said wrapping material (60) in a folded configuration.

8. A sanitary napkin (20) according to Claim 6 or 7 characterised in that said wrapping material (60) is folded about two transversal lines to form an e-fold.
